(19) Europäisches Patentamt
European Patent Office
Office européen des brevets

(11) Publication number: **0 001 503**
**B1**

(12) **EUROPEAN PATENT SPECIFICATION**

(51) Int. Cl.[3]: **A 61 K 31/44,** A 61 K 31/425, A 61 K 31/445, A 61 K 31/60, (A 61 K 31/44, 31/19), (A 61 K 31/44, 31/12), (A 61 K 31/44, 31/405), (A 61 K 31/425, 31/19), (A 61 K 31/425, 31/12), (A 61 K 31/425, 31/405), (A 61 K 31/445, 31/19), (A 61 K 31/445, 31/12), (A 61 K 31/445, 31/405)

(21) Application number: **78300453.4**

(22) Date of filing: **04.10.78**

(54) **Antithrombotic compositions containing benzisothiazolones**

(30) Priority: **08.10.77 GB 41969/77**

(43) Date of publication of application:
**18.04.79 Bulletin 79/08**

(45) Publication of the grant of the European patent:
**07.01.81 Bulletin 81/01**

(84) Designated Contracting States:
**BE CH DE FR GB LU NL SE**

(56) References cited:
**DE - A - 2 652 201**

(73) Proprietor: **BEECHAM GROUP LIMITED**
**Beecham House Great West Road**
**Brentford, Middlesex (GB)**

(72) Inventor: **Nunn, Barbara**
**42 Worcester Road**
**Sutton Surrey (GB)**
**Baggaley, Keith Howard**
**3 Blackstone Hill**
**Redhill Surrey (GB)**

(74) Representative: **Russell, Brian John, et al**
**European Patent Attorney Beecham**
**Pharmaceuticals**
**Yew Tree Bottom Road**
**Epsom Surrey KT18 5XQ (GB)**

**EP 0 001 503 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Antithrombotic compositions containing benzisothiazolones

This invention relates to antithrombotic compositions and in particular to compositions comprising as active ingredients a combination of a benzisothiazolone and an anti-inflammatory agent.

Arterial thrombosis develops initially from the aggregation of blood platelets within the artery. This aggregate may eventually lead to the formation of fibrin and the formation of a consolidated occlusive thrombus. The most widely used therapy for thrombosis is the use of anti-coagulant agents, which influence blood clotting. However, although effective in venous thrombosis, where the thrombus is formed mainly of fibrin, anti-coagulant therapy has no effect on platelet aggregation and has therefore limited effectiveness in arterial thrombosis. It is now accepted that anti-coagulant drugs have little to offer in the treatment of arterial thrombosis.

With the increasing recognition of the primary role of platelets in thrombosis, attention had turned to drugs which are capable of inhibiting the aggregation of platelets.

West German Offenlegungsschrift No. 2652201 discloses a class of benzisothiazolones of formula (I):

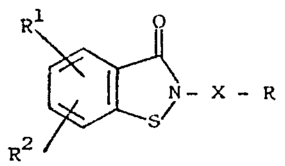

(I)

wherein $R^1$ and $R^2$ are the same or different and each is hydrogen, lower alkyl, lower alkoxy, halo-lower alkyl, nitro, amino, acylamino or halogen, or $R^1$ together with $R^2$ when attached to adjacent carbon atoms represent a $C_3$—$C_6$ alkylene or oxy-($C_1$—$C_3$)-alkyleneoxy moiety; X represents a bond or a straight or branched chain alkylene group having from one to twelve carbon atoms; and R represents a nitrogen-containing heterocyclic ring, or a group of formula —NH.$R^3$ wherein $R^3$ is a nitrogen-containing heterocyclic ring, the groups R and $R^3$ being optionally substituted with a lower alkyl, carboxy or alkoxycarbonyl group; as being effective in inhibiting platelet aggregation.

It has now been found that the activity of the compounds of formula (I) is potentiated by the addition of a non-steroidal anti-inflammatory agent.

The present invention provides a pharmaceutical composition comprising a compound of formula (I) above or a pharmaceutically acceptable acid addition salt thereof characterised in that the composition additionally comprises a non-steroidal anti-inflammatory agent.

One sub-group of compounds within formula (I) is represented by formula (II):

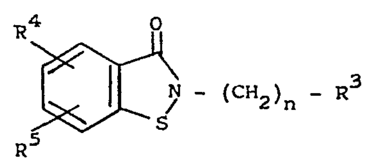

(II)

wherein $R^4$ and $R^5$ are hydrogen, $C_{1-6}$ alkyl or $C_{1-6}$ alkoxy, $R^3$ represents l-pyrrolidinyl, 1-piperidinyl, 3-azabicyclo [3.2.2.]-non-3-yl, morpholino or piperazino, and n represents an integer from 1 to 6. Particular compounds of formula (II) include:

2-[2-(1-piperidyl)ethyl]-1,2-benzisothiazol-3-one;
2-[3-(1-piperidyl)propyl]-1,2-benzisothiazol-3-one;
2-[2-(3-azabicyclo[3.2.2]non-3-yl)ethyl]-5,6-dimethoxy-1,2-benzisothiazol-3-one;
2-[4-(3-azabicyclo[3.2.2]non-3-yl)butyl]-5,6-dimethoxy-1,2-benzisothiazol-3-one.

The benzisothiazolone compounds of formula (I) may be prepared by the methods described in West German O.L.S. no. 2652201.

The anti-inflammatory agents which may be incorporated in the compositions of this invention include the following categories:

A) Indole and indene acetic acids, for example Indomethacin and Sulindac (Registered Trade Mark);

B) Substituted phenyl alkanoic acids and derivatives, for example Ibuprofen, Ketoprofen, fenoprofen, Alclofenac, dichlorofenac, flurbiprofen, piroprofen, suprofen, indoprofen, fenbufen, fenclofenac and compounds of formula (III):

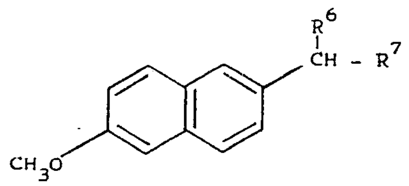

(III)

wherein $R^6$ is hydrogen or methyl, and $R^7$ is a group of formula $—CO_2H$ or $—CH_2COCH_3$, for example naproxen ($R^6 = CH_3$, $R^7 = CO_2H$), (6′-methoxy-2′-napthyl)-acetic acid, and 4-(6′-methoxy-2′-naphthyl)-butan-2-one;

C) N-aryl anthranilic acids such as flufenamic, meclofenamic, niflumic acid, clonixin, and flunixin;
D) Heteroaryl acids, such as tolmetin and prodolic acid;
E) Salicylic acids, for example aspirin (acetylsalicylic acid), flufenisal and diflunisal;
F) Cyclohexyl phenyl acetic acids, such as fenclorac;
G) Other compounds such as
benoxaprofen (see J. Pharm. Pharmac. *29,* 330-336 (1977))
proquazone (see Arch. int. Pharmacodyn. *221,* 122-131 (1976))
piroxicam (see Arzneim-Forsch *26*, No. 7 1300—1303 (1976)).

Other compounds suitable for inclusion in the compositions of this invention are disclosed in "Anti-inflammatory Agents — Chemistry and Pharmacology", *13* numbers I and II, edited by R. A. Scherrer and M. W. Whitehouse, Academic Press (1974).

Particularly suitable anti-inflammatory agents for the compositions of this invention are aspirin (Registered Trade Mark) and indomethacin.

For anti-inflammatory agents which possess a carboxylic acid group in the molecule, the combination of the anti-inflammatory agent and compound of formula (I) may be in the form of a salt. One suitable anti-inflammatory agent for preparing salts with compounds of formula (I) is aspirin. Typical salts include the aspirinate salts of the following compounds:
2-[2-(1-piperidyl)ethyl]-1,2-benzisothiazol-3-one;
2-[3-(1-piperidyl)propyl]-1,2-benzisothiazol-3-one;
2-[2-(3-azabicyclo[3.2.2.]non-3-yl)ethyl]-5,6-dimethoxy-1,2-benzisothiazol-3-one;
2-[4-(3-azabicyclo[3.2.2]non-3-yl)butyl]-5,6-dimethoxy-1,2-benzisothiazol-3-one.

The salts of compounds of formula (I) with carboxylic acid-containing anti-inflammatory agents may be prepared by mixing the two components together, in a suitable solvent.

The compositions of this invention may be formulated for administration by any route, although an oral administration is preferred. The compositions may be in the form of tablets, capsules, powders, granules, lozenges, or liquid preparations, such as oral or sterile parenteral solutions or suspensions.

Tablets and capsules for oral administration may be in the unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol, or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups, or elixirs, or may be presented as dry product for reconstitution with water or other suitable vehicle before use. Such liquid preparations may contain conventional additives such as suspending agents, for example sorbitol, syrup, methyl cellulose, glucose syrup, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl *p*-hydroxybenzoate or sorbic acid, and if desired conventional flavouring or colouring agents. The compounds may also if desired by incorporated in a foodstuff, for example in the form of a biscuit.

For parenteral administration, fluid unit dosage forms are prepared utilizing the compound and a sterile vehicle, water being preferred. The compounds, depending on the vehicle and concentration used, can be either suspended or dissolved in the vehicle. In preparing solutions the compounds can be dissolved in water for injection and filter sterilized before filling into a suitable vial or ampoule and sealing. Advantageously, adjuvants such as a local anaesthetic, preservative and buffering agents can be dissolved in the vehicle. To enhance the stability, the composition can be frozen after filling into the vial and the water removed under vacuum. The dry lyophilized powder is then sealed in the vial. Parenteral suspensions are prepared in substantially the same manner except that the compounds are suspended in the vehicle instead of being dissolved and sterilization cannot be accomplished by filtration. The compounds can be sterilized by exposure to ethylene oxide before suspending in the sterile vehicle. Advantageously, a surfactant or wetting agent is included in the composition to facilitate uniform distribution of the compounds.

The relative molar proportions of the anti-inflammatory agent to the compound of formula (I) may be varied over a wide range depending on the potency of the particular compounds employed. For example, indomethacin is a potent anti-inflammatory agent and should be combined with a compound (I) in the molar ratio of about 1:200. On the other hand aspirin is less potent and when combined with a compound (I) having high inhibition of platelet aggregation activity, the molar ratio of aspirin:compound (I) should be about 2:1. In general the molar ratio of anti-inflammatory to compound (I) will be from 10:1 to 1:10 suitably from 5:1 to 1:5. It will usually be preferable to employ more compound (I) than anti-inflammatory agent.

The compositions may contain from 0.1% to 99% by weight, preferably from 10% to 60% by weight, of the active material, depending on the method of administration. Where the compositions comprise dosage units, each unit will preferably contain from 1—500 mg of the active ingredient.

The dosage employed for adult treatment will of course depend on the dose-response characteristics of the particular active ingredient, and also on the blood volume and condition of the patient, but will normally be in the range of 0.01 to 30 mg/kg/day depending on the route and frequency of administration. The preferred dose is 10 to 500 mg., orally 1 to 3 times a day for an adult human.

The compositions of the invention are useful for administration to humans and animals to prevent clot formation for example after surgery to prevent postoperative thrombosis; in geriatric patients to prevent transient cerebral ischemic attacks; and long-term prophylaxis following myocardial infarcts and strokes.

*Biological Data*

The structures of four compounds tested are as follows:

Compound A:

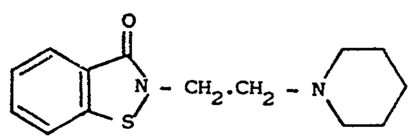

Compound B:

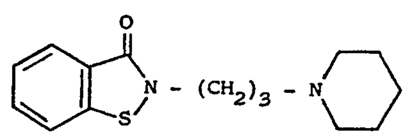

Compound C:

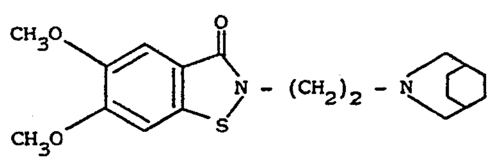

Compound D:

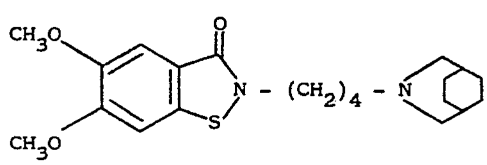

These compounds were tested for inhibition of platelet aggregation alone and in combination with certain anti-inflammatory agents, in the guinea pig *ex vivo* by the following method:

Ten male guinea pigs weighing 250—300 g were orally dosed 1% methyl cellulose (5 ml/kg) in which the compound under test was suspended. Ten control animals were given methyl cellulose alone. Two hours later, each animal was killed and 4.5 ml blood drawn from the inferior vena cava into 0.5 ml trisodium citrate dihydrate. Platelet-rich-plasma (PRP) was prepared from each blood sample by centrifigation at 450 g to 5 min. The platelet concentration in each sample of PRP was adjusted with autologous platelet-poor-plasma to give a count of 500,000 platlets/$\mu$l PRP. Aggregation was measured turbidometrically (G.V.R. Born, 1962, Nature, *194*, 927—929) at 39° using a Bryston aggregometer coupled to a penrecorder. The concentration of callogen producing approximately 50%

maximal aggregation and the concentration of ADP producing first-phase aggregation were compared in PRP samples from control and drug treated animals.

In the tables the dose ratio represents the ratio of the concentration of aggregating agent to cause aggregation in PRP from drug-treated animals to the concentration of aggregating agent to cause aggregation in PRP from control animals. The "% inhibition of ADP" column represents the % inhibition of aggregation caused by ADP, and the asterisks beside the figures mean that they are statistically significantly different from the control results.

RESULTS

1) Aspirin and compound A (2-[2-(1-piperidyl)ethyl]-1,2-benzisothiazol-3-one)

TABLE 1

| Compound | Dose p.o. mmol (mg) /kg | Dose-ratio | | % Inhibition of ADP | |
|---|---|---|---|---|---|
| | | vs. Coll. | vs. ADP | 0.5 μM | 1 μM |
| Aspirin | 0.15 (27) | 2.2* | 1.3 | 16 | 0 |
| Compound A | 0.08 (20) | 8.3* | 1.4* | 26 | 0 |
| Aspirin + Compound A | 0.15 (27) + 0.08 (20) | >29* | 3.5* | 85* | 57* |

2) Aspirin and compound B (2-[3-(1-piperidyl)propyl]-1,2-benzisothiazol-3-one)

TABLE 2

| Compound | Dose p.o. mmol (mg) /kg | Dose-ratio | | % Inhibition of ADP |
|---|---|---|---|---|
| | | vs. Coll. | vs. ADP | (0.5 μM) |
| Aspirin | 0.15 (27) | 2,2* | 1.3 | 0 |
| Compound B | 0.08 (22) | 9.0* | 1.7* | 57* |
| Aspirin + Compound B | 0.15 (27) + 0.08 (22) | >40* | 7.5* | 100* |

3) Indomethacin and compound B

TABLE 3

| Compound | Dose p o. mmol (mg) /kg | Dose-ratio vs. Coll. | Dose-ratio vs ADP | % Inhibition of ADP (0.5$\mu$M) |
|---|---|---|---|---|
| Indomethacin | 0.003 (1) | 1.7* | 1.1 | 0 |
| Compound B | 0.08 (22) | 9.0* | 1.7* | 57* |
| Indomethacin + compound B | 0.003 (1) + 0.08 (22) | >18* | 6.3* | 79* |

4) Aspirin and compound C (2-[2-(3-azabicyclo[3.2.2] non-3-yl)ethyl]-5,6-dimethoxy-1,2-benzisothiazol-3-one)

TABLE 4

| Compound | Dose p.o. mmol (mg) /kg | Dose ratio vs. Coll. | Dose ratio vs. ADP | % Inhibition of ADP (0.5$\mu$M) |
|---|---|---|---|---|
| Aspirin | 0.15 (27) | 2.2* | 1.3 | 0 |
| Compound C | 0.6 (217) | >13.9* | 3.3* | 92* |
| Aspirin + Compound C | 0.15 (27) + 0.6 (217) | >36* | 4.9* | 92* |

5) Aspirin and compound D (2-[4-(3-azabicyclo[3.2.2] non-3-yl) butyl]-5,6-dimethoxy-1,2-benzisothiazol-3-one)

TABLE 5

| Compound | Dose p.o. mmol (mg) /kg | Dose ratio vs. Coll. | Dose ratio vs. ADP | % Inhibition of ADP (0.5$\mu$M) |
|---|---|---|---|---|
| Aspirin | 0.15 (27) | 2.2* | 1.3 | 0 |
| Compound D | 0.08 (31) | 5.4* | 2.6* | 86* |
| Aspirin + Compound D | 0.08 (31) + 0.15 (27) | >20.4* | 4.3* | 100* |

6) Aspirinate salt of compound D

TABLE 6

| Compound | Dose p.o. mmol (mg) /kg | Dose ratio vs Coll. | Dose ratio vs. ADP | % Inhibition of ADP (0.5$\mu$M) |
|---|---|---|---|---|
| Aspirin | 0.15 (27) | 2.2* | 1.3 | 0 |
| Compound D | 0.15 (59) | >15.4* | 3.2* | 61* |
| Compound D Aspirinate Salt | 0.15 (88) | >57* | 4.6* | 91* |

Example 1

Aspirin salt of 2-(4-(3-azabicyclo[3.2.2.]non-3-yl)butyl)-5,6-dimethoxy-1,2-benzisothiazol-3-one

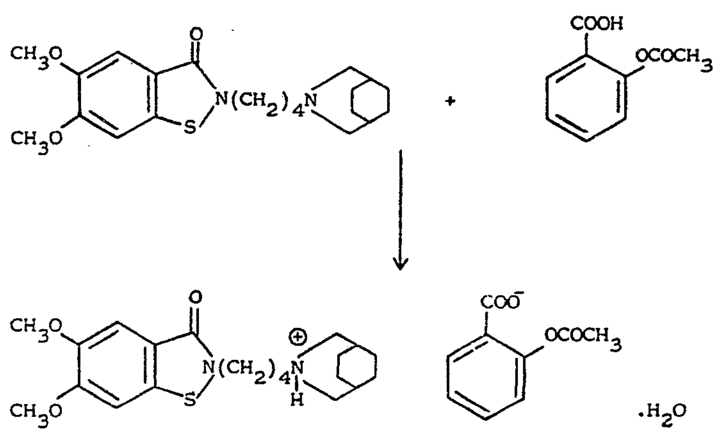

A mixture of 2-(4-(3-azabicyclo[3.2.2.]non-3-yl)butyl)-5,6-dimethoxy-1,2-benzisothiazol-3-one (811.2 mg., 2.077 mmole) and aspirin (375.0 mg., 2.081 mmole) was dissolved in propan-2-ol (3.5 ml) by gentle warming. Di-isopropyl ether (10 ml) was added and the solution was filtered. More di-isopropyl ether (8 ml) was added to the filtrate and the solution was allowed to stand at room temperature for 3 days. No attempt was made to exclude moisture from the reaction mixture. The aspirin salt (monohydrate) of the benzisothiazolone slowly separated as colourless prisms (1.05 g., 86%, m.p. 107—110° (softens at 96°).

Example 2

A mixture of 25 g of 2-[2-(1-piperidyl)ethyl]-1,2-benzisothiazol-3-one and 12.5 g of aspirin are blended together and used to fill 500 gelatin capsules, each capsule containing 50 mg of the benzisothiazolone and 25 mg of aspirin.

Examples 3—7

A similar procedure is followed for the following cominations.

| Example No. | |
|---|---|
| 3 | Aspirin: 25 g<br>Compound A: 20 g |
| 4 | Aspirin: 25 g<br>Compound B: 20 g |
| 5 | Indomethacin: 1 g<br>Compound B: 20 g |
| 6 | Aspirin: 25 g<br>Compound C: 200 g |
| 7 | Aspirin: 25 g<br>Compound D: 30 g |

## Claims

1. A pharmaceutical composition comprising a benzisothiazolone compound of formula (I) or a pharmaceutically acceptable acid addition salt thereof:

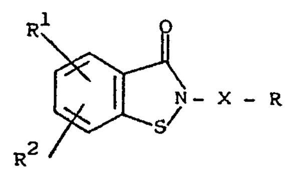

(I)

wherein $R^1$ and $R^2$ are the same or different and each is hydrogen. $C_1$ to $C_6$ alkyl, $C_1$ to $C_6$ alkoxy, halo $C_1$ to $C_6$ alkyl, nitro, amino, acylamino or halogen, or $R^1$ together with $R^2$ when attached to adjacent carbon atoms represent a $C_3$—$C_6$ alkylene or oxy-($C_1$_$C_3$)-alkyleneoxy moiety; X represents a bond or a straight or branched chain alkylene group having from one to twelve carbon atoms; and R represents a nitrogen-containing heterocyclic ring, or a group of formula —NH.$R^3$ wherein $R^3$ is a nitrogen-containing heterocyclic ring, the groups R and $R^3$ being optionally substituted with a $C_1$ to $C_6$ alkyl, carboxy or alkoxycarbonyl group characterised in that the composition additionally comprises a non-steroidal anti-inflammatory agent.

2. A composition as claimed in claim 1 wherein the group R in the compound of formula (I) is 2-pyridyl, 1-pyrrolidinyl, 1-piperidinyl, or 3-azabicyclo[3.2.2]-non-3-yl.

3. A composition as claimed in claim 1 wherein the benziothiazolone compound has formula (II):

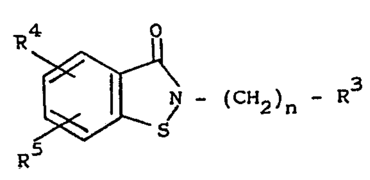

(II)

wherein $R^4$ and $R^5$ are hydrogen, $C_{1-6}$ alkyl or $C_{1-6}$ alkoxy, $R^3$ represents 1-pyrrolidinyl, 1-piperidinyl, 3-azabicyclo[3.2.2.]-non-3-yl, morpholino or piperazino, and n represents an integer from 1 to 6.

4. A composition as claimed in claim 3 wherein the groups $R^4$ and $R^5$ are hydrogen or methoxy.

5. A composition as claimed in claim 4 wherein the benzisothiazolone compound of formula (I) is selected from:

2-[2-(1-piperidyl)ethyl]-1,2-benzisothiazol-3-one;
2-[3-(1-piperidyl)propyl]-1,3-benzisothiazol-3-one;
2-[2-(3-azabicyclo[3.2.2]non-3-yl)ethyl]-5,6-dimethoxy-1,2-benzisothiazol-3-one;
2-[4-(3-azabicyclo[3.2.2]non-3-yl)butyl]-5,6-dimethoxy-1,2-benzisothiazol-3-one.

6. A composition as claimed in any one of claims 1 to 5 wherein the anti-inflammatory agent is a compound of formula (III):

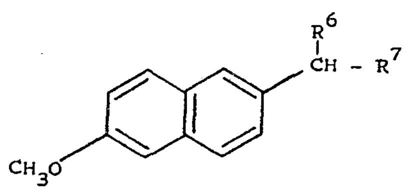

(III)

wherein $R^6$ is hydrogen or methyl, and $R^7$ is a group of formula $—CO_2H$ or $—CH_2COCH_3$.

7. A composition as claimed in any one of claims 1 to 5 wherein the anti-inflammatory agent is acetylsalicylic acid or indomethacin.

8. A salt of a benzisothiazolone compound of formula (I) as defined in claim 1 with a non-steroidal anti-inflammatory agent having a carboxylic acid group in the molecule.

9. The acetylsalicylate salt of a benzisothiazolone compound of formula (I) as defined in claim 1.

10. A salt as claimed in claim 9 wherein the benzisothiazolone compound is a compound of formula (II) as defined in claim 3.

**Patentansprüche**

1. Pharmazeutische Zusammensetzungen, gekennzeichnet durch einen Gehalt an einer Benzisothiazolon-Verbindung der allgemeinen Formel (I) oder deren pharmakologisch verträgliches Säureadditionssalz

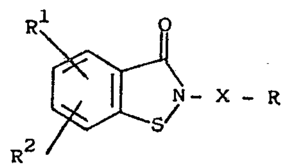

(I)

in der $R^1$ und $R^2$ gleich oder verschieden sind und jeweils ein Wasserstoffatom, einen Alkylrest mit 1 bis 6 Kohlenstoffatomen, einen Alkoxyrest mit 1 bis 6 Kohlenstoffatomen, einen Halogenalkylrest mit 1 bis 6 Kohlenstoffatomen, die Nitrogruppe, die Aminogruppe, einen Acylaminorest oder ein Halogenatom bedeuten oder $R^1$ zusammen mit $R^2$, wenn sie an benachbarte Kohlenstoffatome gebunden sind, einen Alkylenrest mit 3 bis 6 Kohlenstoffatomen oder einen Oxyalkylenoxy-Rest mit 1 bis 3 Kohlenstoffatomen bedeuten, X eine Bindung oder ein geradkettiger oder verzweigtkettiger Alkylenrest mit 1 bis 12 Kohlenstoffatomen ist und R für einen Stickstoff enthaltenden heterocyclischen Ring oder einen Rest der allgemeinen Formel $—NH.R^3$ steht, wobei $R^3$ ein Stickstoff enthaltender heterocyclischer Ring ist, und in denen die Reste R und $R^3$ gegebenenfalls mit Alkylresten mit 1 bis 6 Kohlenstoffatomen, einem Carboxy- oder einem Alkoxycarbonylrest substituiert sind, dadurch gekennzeichnet, daß die Zubereitung zusätzlich ein nicht-steroides entzündungshemmendes Mittel enthält.

2. Pharmazeutische Zubereitungen nach Anspruch 1, bei denen der Rest R bei der Verbindung der allgemeinen Formel (I) die 2-Pyridyl-, 1-Pyrrolidinyl-, 1-Piperidinyl- oder 3-Azabicyclo[3.2.2] nonyl-3-yl-Gruppe ist.

3. Pharmazeutische Zubereitungen nach Anspruch 1, wobei die Benzisothiazolon-Verbindung die allgemeine Formel (II) aufweist

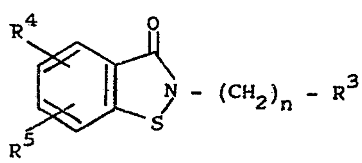

(II)

in der $R^4$ und $R^5$ Wasserstoffatome, Alkylreste mit 1 bis 6 Kohlenstoffatomen oder Alkoxyreste mit 1 bis 6 Kohlenstoffatomen sind, $R^3$ die 1-Pyrrolidinyl-, 1-Piperidinyl-, 3-Azabicyclo[3.2.2]non-3-yl-, Morpholino; oder Piperazino-Gruppe bedeutet und n eine ganze Zahl von 1 bis 6 ist.

4. Pharmazeutische Zubereitungen nach Anspruch 3, wobei die Reste $R^4$ und $R^5$ Wasserstoffatome oder Methoxygruppen sind.

5. Pharmazeutische Zubereitungen nach Anspruch 4, wobei die Benzisothiazolon-Verbindung der allgemeinen Formel (I) ausgewählt ist aus
2-[2-(1-Piperidyl)-äthyl]-1,2-benzisothiazol-3-on;
2-[3-(1-Piperidyl)-propyl]-1,3-benzisothiazol-3-on;
2-[2-(3-Azabicyclo[3.2.2]non-3-yl)-äthyl]-5,6-dimethoxy-1,2-benzisothiazol-3-on;
2-4[4-(3-Azabicyclo[3.2.2]non-3-yl)-butyl]-5,6-dimethoxy-1,2-benzisothiazol-3-on.

6. Pharmazeutische Zusammensetzungen nach einem der Ansprüche 1 bis 5, dadurch gekennzeichnet, daß das entzündungshemmende Mittel eine Verbindung der allgemeinen Formel (III) ist

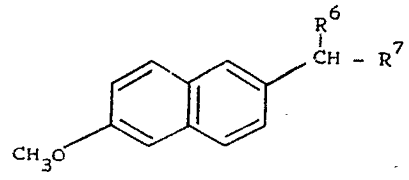

(III)

in der $R^6$ ein Wasserstoffatom oder die Methylgruppe ist und $R^7$ eine Gruppe der Formel $—CO_2H$ oder $—CH_2COCH_3$ bedeutet.

7. Pharmazeutische Zusammensetzungen nach einem der Ansprüche 1 bis 5, dadurch gekennzeichnet, daß das entzündungshemmende Mittel Acetylsalicylsäure oder Indomethacin ist.

8. Ein Salz einer Benzisothiazolon-Verbindung der allgemeinen Formel (I), wie in Anspruch 1 definiert, mit einem nichtsteroiden entzündungshemmenden Mittel, das eine Carboxylgruppe im Molekül aufweist.

9. Das Acetylsalicylsäuresalz einer Benzisothaizolon-Verbindung der allgemeinen Formel (I), wie sie in Anspruch 1 definiert ist.

10. Ein Salz nach Anspruch 9, dadurch gekennzeichnet, daß die Benzisothiazolon-Verbindung eine Verbindung der allgemeinen Formel (II) ist, wie sie in Anspruch 3 definiert ist.

**Revendications**

1. Composition pharmaceutique comprenant un dérivé de benzisothiazolone de formule (I) ou un sel d'addition avec un acide pharmaceutiquement acceptable de celui-ci:

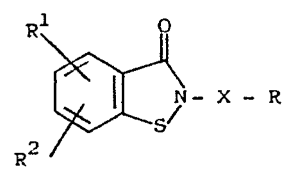

(I)

où $R^1$ et $R^2$ sont identiques ou différents et chacun de ces radicaux représente de l'hydrogène, un alcoyle en $C_1$ à $C_6$, un alcoxy en $C_1$ à $C_6$, un halogénoalcoyle en $C_1$ à $C_6$, un groupe nitro, amino, acylamino ou un halogène, ou bien $R_1$ représente avec $R_2$, lorsqu'ils sont fixés sur des atomes de carbone adjacents, un groupe alcoylène en $C_3—C_6$ ou un groupe oxyalcoylèneoxy en $C_1—C_3$; X représente une liaison ou un groupe alcoylène à chaîne droite ou ramifiée ayant de 1 à 12 atomes de carbone; et R représente un groupe hétérocyclique contenant de l'azote ou un groupe de formule $—NH.R^3$, où $R^3$ est un groupe hétérocyclique contenant de l'azote, les groupes R et $R^3$ étant facultativement substitués par un groupe alcoyle en $C_1$ à $C_6$, un groùpe carboxy ou alcoxy-carbonyle, cette composition étant caractérisée en ce qu'elle renferme, en outre, un agent anti-inflammatoire non stéroïdien.

2. Composition suivant la revendication 1, caractérisée en ce que le groupe R dans le composé de formule (I) est un groupe 2-pyridyle, 1-pyrrolidinyle, 1-pipéridinyle ou 3-azabicyclo [3.2.2.]non-3-yle.

3. Composition suivant la revendication 1, caractérisée en ce que le dérivé de benzisothiazolone présente la formule (II):

(Voir formule page suivante)

où $R^4$ et $R^5$ représentent de l'hydrogène, un groupe alcoyle en $C_{1-6}$ ou alcoxy en $C_{1-6}$, $R^3$ représente un groupe 1-pyrrolidinyle, 1-pipéridinyle, 3-azabicyclo[3.2.2.]-non-3-yle,

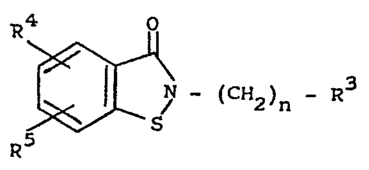

(II)

morpholino ou pipérazino et n représente un numbre entier de 1 à 6.

4. Composition suivant la revendication 3, caractérisée en ce que les groupes $R^4$ et $R^5$ représentent de l'hydrogène ou un méthoxy.

5. Composition suivant la revendication 4, caractérisée en ce que le dérivé de benzisothiazolone de formule (I) est choisi parmi:
la 2-[2-(1-pipéridyl) éthyl]-1,2-benzisothiazol-3-one;
la 2-[3-(1-pipéridyl)propyl]-1,3-benzisothiazol-3-one;
la 2-[2-(3-azabicyclo[3.2.2.]non-3-yl)éthyl]-5,6-diméthoxy-1,2-benzisothiazol-3-one;
la 2,4[4-(3-azabicyclo[3.2.2.]non-3-yl)butyl]-5,6-diméthoxy-1,2-benzisothiazol-3-one.

6. Composition suivant l'une des revendications 1 à 5, caractérisée en ce que l'agent anti-inflammatoire est un composé de formule (III):

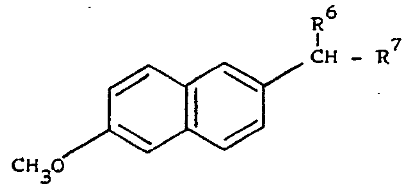

(III)

où $R^6$ est de l'hydrogène ou un méthyle et $R^7$ est un groupe de formule $—CO_2H$ ou $—CH_2COCH_3$.

7. Composition suivant l'une quelconque des revendications 1 à 5, caractérisée en ce que l'agent anti-inflammatoire est de l'aspirine ou de l'indométhacine.

8. Sel d'un dérivé de benzisothiazoline de formule (I) suivant la revendication 1, avec un agent anti-inflammatoire non stéroïdien ayant un groupe carboxylique dans la molécule.

9. Aspirinate d'un dérivé de benzisothiazolone de formule (I) tel que défini dans la revendication 1.

10. Sel suivant la revendication 9, caractérisé en ce que le dérivé de benzisothiazolone est un composé de formule (II) tel que défini dans la revendication 3.